# EUROPEAN PATENT APPLICATION

(11) **EP 2 901 920 A1**
(43) Date of publication of application: **05.08.2015**
(21) Application number: 12885753.9
(22) Date of filing: 25.09.2012
(51) Int. Cl.: A61B 5/00, G01D 9/00, G04G 5/00, G04G 99/00

(54) **BIOLOGICAL INFORMATION PROCESSING SYSTEM, BIOLOGICAL INFORMATION MEASUREMENT DEVICE, CONTROL DEVICE, METHOD FOR CONTROLLING THESE, AND STORAGE MEDIUM**

(71) Applicant: Terumo Kabushiki Kaisha, Tokyo 151-0072 (JP)
(72) Inventor: ARATANI, Tooru, Tokyo 103-0028 (JP)
(74) Representative: Casalonga
(86) International application number: PCT/JP2012/006083
(87) International publication number: WO 2014/049633

(57) **Abstract**

A biological information processing system includes a biological information measurement device that measures biological information on a subject and a control device that manages the biological information. The biological information measurement device includes a storage section and a data transmission section. The storage section relates the biological information, measurement date and time information, and flag information to one another, and stores these pieces of information as measurement data; the measurement date and time information indicates a date and time when the biological information is measured, and the flag information indicates whether the measurement date and time is a provisional date and time when a clock is not adjusted yet. The data transmission section transmits the measurement data and measurement-side clock information possessed by the biological information measurement device to the control device. The control device includes an analysis section, a data correction section and a recording section. The analysis section analyzes the flag information contained in the measurement data that has been received from the biological information measurement device. When the analysis conducted by the analysis section shows that the measurement date and time is the provisional date and time, the data correction section corrects the measurement date and time information on the basis of a time discrepancy between the measurement-side clock information and control-side clock information possessed by the control device. The recording section relates the biological information to corrected measurement date and time information corrected by the data correction section, and records both pieces of information as corrected measurement data.

## Description

### Technical Field

The present invention relates to a biological information processing system, a biological information measurement device, a control device, a method of controlling them, and a storage medium. More specifically, the present invention relates to a biological information processing technique through which a control device acquires and stores biological information measured by a biological information measurement device.

### Background Art

In current medical practice, pulse oximeters, clinical thermometers, blood glucose meters, activity meters and other biological information measurement devices are used to measure biological information on subjects. Then, control devices, such as personal computers, acquire the measurement result and manage the biological information on subjects. A pulse oximeter as described above is a device that measures arterial oxygen saturation (SpO2) (refer to Patent Literature 1).

Such a biological information measurement device stores pieces of biological information measured to which their measurement dates and times are related. Accordingly, the control device acquires pieces of biological information as well as their measurement dates and times, thereby managing the biological information on subjects on a time-series basis.

### Citation List

### Patent Literature

Patent Literature 1: JP 05-320038 A

### Summary of Invention

### Technical Problem

If a biological information measurement device is not equipped with means for setting clock information or a function of maintaining it, the discrepancy may arise between the clock information retained in the biological information measurement device and right clock information. This is because, for example, when a measurer replaces the battery of the biological information measurement device and powers it up again, provisional date and time information is set.

Thus, when a measurer measures biological information on a subject by using a biological information measurement device, there are cases where the measured biological information and its measurement date and time are stored while not related to each other correctly. More specifically, a control device may acquire biological information to which a wrong measurement date and time differing from an actual date and time is related. Therefore, there has been a problem in that it is difficult to manage biological information on subjects appropriately.

In light of the problem described above, an object of the present invention is to provide a technique for enabling appropriate management of biological information on subjects.

### Solution to Problem

A biological information processing system according to the present invention which accomplishes the foregoing object is a biological information processing system that includes a biological information measurement device that measures biological information on a subject and a control device that manages the biological information. The biological information measurement device includes: storage means for relating the biological information, measurement date and time information, and flag information to one another and storing these pieces of information as measurement data; and data transmission means for transmitting the measurement data and measurement-side clock information possessed by the biological information measurement device to the control device. The measurement date and time information indicates a date and time when the biological information is measured; the flag information indicates whether the measurement date and time is a provisional date and time when a clock is not adjusted yet. The control device includes: analysis means for analyzing the flag information contained in the measurement data, the measurement data being received from the biological information measurement device; data correction means for, when the analysis conducted by the analysis means shows that the measurement date and time is the provisional date and time, correcting the measurement date and time information on the basis of a time discrepancy between the measurement-side clock information and control-side clock information possessed by the control device; and recording means for relating the biological information to corrected measurement date and time information corrected by the data correction means, and recording both pieces of information as corrected measurement data.

### Advantageous Effects of Invention

The present invention enables appropriate management of biological information on subjects.

Other features and advantages of the present invention will be apparent from the following description that makes reference to the accompanying drawings. In the accompanying drawings, the same reference numbers are given to identical or similar configurations.

### Brief Description of Drawings

The accompanying drawings are included in the description and make up a part thereof. In addition, the drawings illustrate embodiments of the present invention and are used together with the recitation of the embodiments to explain the principle of the present invention.
Fig. 1 is a view illustrating an exterior configuration of a biological information processing system 100 according to a first embodiment.
Fig. 2 is a view illustrating a hardware configuration of the control device 110 according to the first embodiment.
Fig. 3 is a functional block diagram of the control device 110 according to the first embodiment.
Fig. 4 is a view illustrating an exterior configuration of the pulse oximeter 131 according to the first embodiment and a mode of use thereof.
Fig. 5 is a view illustrating a hardware configuration of the control device 110 according to the first embodiment.
Fig. 6 is a functional block diagram of the pulse oximeter 131 according to the first embodiment.
Fig. 7A is a sequence diagram describing procedures of a process performed by the biological information processing system 100 according to the first embodiment.
Fig. 7B is a sequence diagram describing procedures of the process performed by the biological information processing system 100 according to the first embodiment.
Fig. 8 is a view illustrating an exemplary configuration of measurement data according to the first embodiment.
Fig. 9A is a sequence diagram describing procedures of a process performed by a biological information processing system 100 according to a second embodiment.
Fig. 9B is a sequence diagram describing procedures of the process performed by the biological information processing system 100 according to the second embodiment.
Fig. 10A is a sequence diagram describing procedures of a process performed by a biological information processing system 100 according to a third embodiment.
Fig. 10B is a sequence diagram describing procedures of the process performed by the biological information processing system 100 according to the third embodiment.
Fig. 11A is a sequence diagram describing procedures of a process performed by a biological information processing system 100 according to a fourth embodiment.
Fig. 11B is a sequence diagram describing procedures of the process performed by the biological information processing system 100 according to the fourth embodiment.

### Description of Embodiments

Embodiments of the present invention will be described below with reference to the drawings. Embodiments that will be described below are preferred, specific examples of the present invention, so various limitations are placed on them. However, the scope of the present invention is not limited to these aspects unless any specific limitation on the present invention is described in the following explanation.

### [First Embodiment]

### <1. Exterior Configuration of Biological Information Processing System>

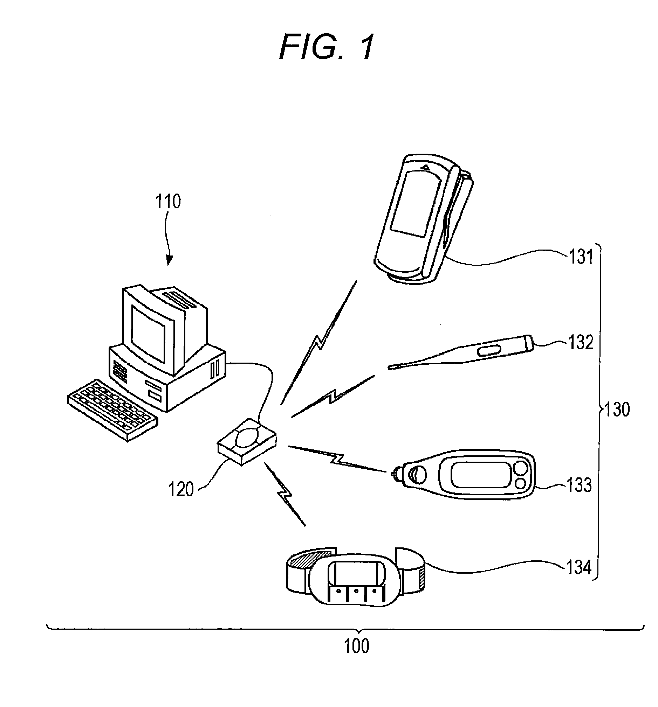

Fig. 1 is a view illustrating an exterior configuration of a biological information processing system 100 according to a first embodiment. In Fig. 1, the biological information processing system 100 includes a control device 110, a proximity communication unit 120 and a biological information measurement device 130. Specifically, the biological information measurement device 130 is one of a pulse oximeter 131, a clinical thermometer 132, a blood glucose meter 133, an activity meter 134, and any other given device that can measure biological information on subjects.

The control device 110 acquires and analyzes clock information (measurement-side clock information) in the biological information measurement device 130 and measurement data measured thereby. As a result of the analysis, the control device 110 corrects as appropriate the measurement data (a measurement date and time in the measurement data which have been stored in accordance with the clock information in the biological information measurement device 130); this correction is based on the time discrepancy between the clock information in the biological information measurement device 130 and the clock information (control-side clock information) in the control device 110 itself. Then, the control device 110 records the corrected measurement data.

The proximity communication unit 120 conducts near field communication (NFC). The NFC refers to a technique of near field communication conducted at a frequency of 13.56 MHz and at a communication distance of about 10 cm.

The biological information measurement device 130 measures biological information on subjects. Then, the biological information measurement device 130 stores the measured biological information and a measurement date and time in the clock information in the biological information measurement device 130 in relation to each other. In the case of the pulse oximeter 131, it measures the arterial oxygen saturations (SpO2) of subjects. In the case of the clinical thermometer 132, it measures the body temperatures of subjects. In the case of the blood glucose meter 133, it measures the blood glucoses of subjects. In the case of the activity meter 134, it measures the amounts of activities of subjects.

A user places the biological information measurement device 130 close to or on the proximity communication unit 120 whereby data communication is made possible between the control device 110 and the biological information measurement device 130. Various types of data can be exchanged therebetween.

The proximity communication unit 120 may be built in the control device 110. The data communication between the control device 110 and the biological information measurement device 130 is not limited to the near field communication and may be conducted via another wireless or wired connection.

### <2. Hardware Configuration and Functional Block Configuration of Control Device>

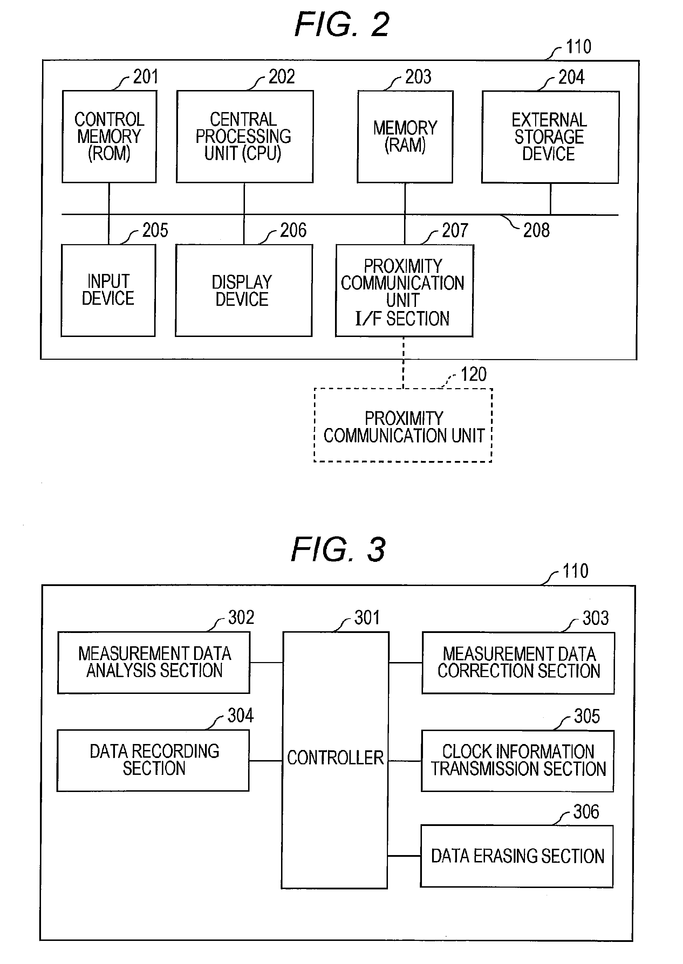

Next, a description will be given of a configuration of the control device 110 that is a component of the biological information processing system 100. Fig. 2 is a view illustrating a hardware configuration of the control device 110. The control device 110 is implemented, for example, using a personal computer and includes a control memory (ROM) 201, a central processing unit (CPU) 202, a memory (RAM) 203, an external storage device 204, an input device 205, a display device 206 and a proximity communication unit I/F section 207. These components are interconnected by a bus 208.

The central processing unit (CPU) 202 reads a program stored in the external storage device 204 (storage medium) via the bus 208, then enters it in the memory (RAM) 203 and runs the entered program. Furthermore, the central processing unit (CPU) 202 reads a program stored in the control memory (ROM) 201 (storage medium) and runs it. In this way, the central processing unit (CPU) 202 controls the operation of the control device 110.

The external storage device 204 stores the measurement data received from the biological information measurement device 130 and the clock information in the biological information measurement device 130. The input device 205 is implemented, for example, using a keyboard and receives user's operations from the control device 110. The display device 206 is implemented, for example, using a liquid crystal display and shows various types of information. The proximity communication unit I/F section 207 serves as an interface to be connected to the proximity communication unit 120.

Fig. 3 is a functional block diagram of the control device 110. The control device 110 includes a controller 301, a measurement data analysis section 302, a measurement data correction section 303, a data recording section 304, a clock information transmission section 305 and a data erasing section 306.

The controller 301 controls the operations of the components connected thereto. The measurement data analysis section 302 analyzes the measurement data received from the biological information measurement device 130 through the near field communication. The measurement data contains the biological information on subjects, the measurement date and time information, and flag information. The biological information on subjects is measured by the biological information measurement device 130. The measurement date and time information concerns the biological information and is based on the clock information in the biological information measurement device 130. The flag information indicates whether the measurement date and time is a provisional date and time before the clock is adjusted. The expression "a case where a measurement date and time is a provisional date and time" refers to a case where clock information in the biological information measurement device 130 is reset to a provisional date and time such as 12:00, for example, when the battery of the biological information measurement device 130 is replaced and not yet modified to right clock information. Specifically, the measurement data analysis section 302 analyzes the flag information contained in the measurement data.

If the analysis conducted by the measurement data analysis section 302 shows that the measurement date and time is a provisional date and time, the measurement data correction section 303 corrects the measurement date and time information contained in the measurement data so that it becomes a right measurement date and time. This correction is based on the time discrepancy between the clock information in the control device 110 and the clock information in the biological information measurement device 130 which has been received from the biological information measurement device 130 through the near field communication. The data recording section 304 relates the biological information contained in the measurement data to the corrected measurement date and time information that the measurement data correction section 303 has corrected, and then records both pieces of information as corrected measurement data.

As described above, if the clock information in the biological information measurement device 130 is reset and displaced from right clock information, the control device 110 corrects the measurement date and time information contained in the measurement data. This enables the measurement result of the biological information measurement device 130 to be managed appropriately.

The clock information transmission section 305 transmits the clock information in the control device 110 to the biological information measurement device 130 through the near field communication. The biological information measurement device 130 can modify the clock information of the biological information measurement device 130 on the basis of the clock information in the control device 110 which has been received from the control device 110. The data erasing section 306 deletes unnecessary measurement data.

### <3. Exterior Configuration of Biological Information Measurement Device and Mode of Use thereof>

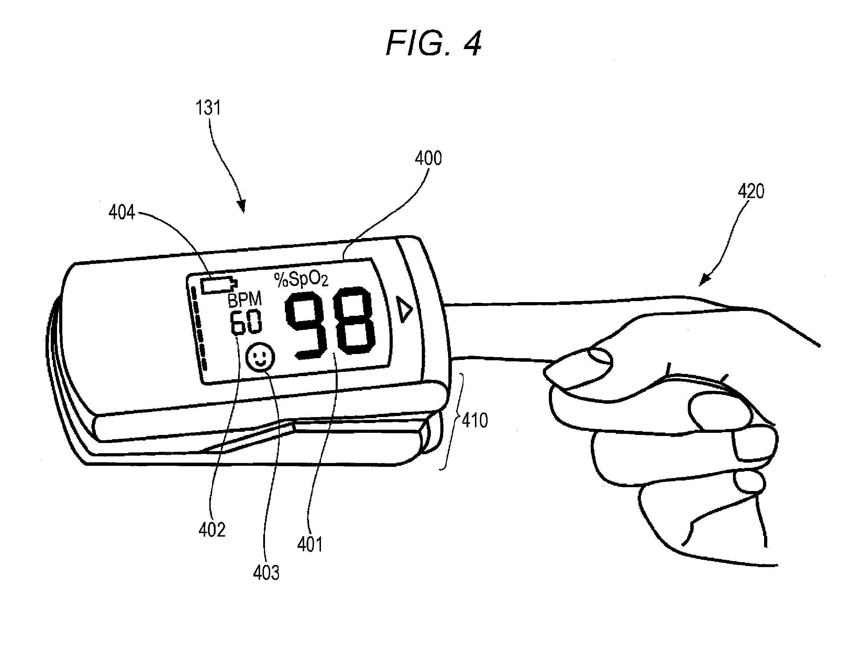

The pulse oximeter 131 illustrated in Fig. 1 will be described below as an exemplary biological information measurement device 130 that is a component of the biological information processing system 100. Fig. 4 is a view illustrating an exterior configuration of the pulse oximeter 131 and a mode of use thereof.

A pulse oximeter is a medical instrument that has a probe to be attached to a fingertip or the like and measures arterial oxygen saturation (SpO2) and a pulse rate. The arterial oxygen saturation (SpO2) indicates the degree to which oxygen (02) is bonded to hemoglobin (Hb) contained in arterial blood. For example, if the arterial oxygen saturation at rest is equal to or more than 93%, the cardiopulmonary function can be determined to be normal. The probe is equipped with a light emitting section and a light receiving section; the light emitting section emits red light and infrared light, and the light receiving section detects light that passes through or reflected by the fingertip or the like. The pulse oximeter can measure the arterial oxygen saturation (SpO2) by analyzing the difference in absorbance between the red light and infrared light which depends on the bond between oxygen and hemoglobin contained in blood. In addition, the pulse oximeter can calculate a pulse rate from a beating, pulse wave component contained in a detected pulse.

As illustrated in Fig. 4, the pulse oximeter 131 is equipped with a display section 400. This display section 400 shows an arterial oxygen saturation (SpO2) measurement 401, a pulse rate measurement 402, a display mode 403 that indicates the health condition of a subject which is determined from the measurement results, and a remaining battery level 404. When a subject's hand 420 such as a forefinger is inserted into an insertion unit 410 and then a measurement start switch (not illustrated) is pushed down, the pulse oximeter 131 measures the arterial oxygen saturation (SpO2). Alternatively, the pulse oximeter 131 may start measuring it in response to the detection of the insertion of the subject's hand 420.

The pulse oximeter 131 stores the measured arterial oxygen saturation (SpO2), the measurement date and time information regarding this arterial oxygen saturation (SpO2), and flag information in relation to one another; the measurement date and time information is based on the clock information in the pulse oximeter 131, and the flag information indicates whether the measurement date and time is a provisional date and time.

### <4. Hardware Configuration and Functional Block Configuration of Biological Information Measurement Device>

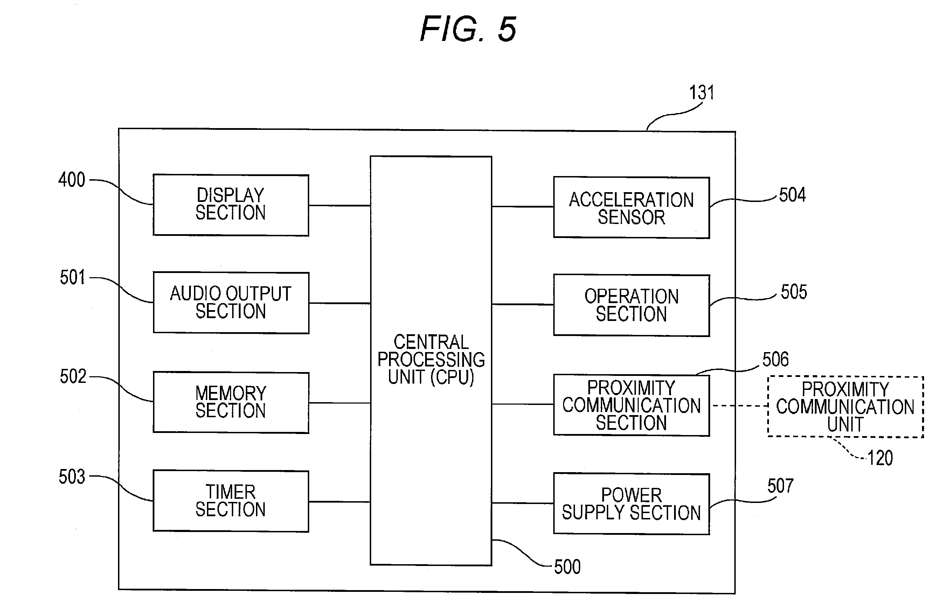

Next, a configuration of the pulse oximeter 131 will be described below as an exemplary biological information measurement device 130. Fig. 5 is a view illustrating a hardware configuration of the control device 110. The same reference characters are given to the components having already been described.

The pulse oximeter 131 includes the display section 400, a central processing unit (CPU) 500, an audio output section 501, a memory section 502, a timer section 503, an acceleration sensor 504, an operation section 505, a proximity communication unit 506 and a power supply section 507.

The central processing unit (CPU) 500 reads and executes a program stored in the memory section 502, thereby controlling an operation of the pulse oximeter 131. The audio output section 501 emits a beep sound that indicates the reception of an input instruction via the operation section 505 or gives an alarm when a predetermined condition is satisfied. The memory section 502 stores data regarding measured arterial oxygen saturation (SpO2) and pulse rates, and the like.

The timer section 503 outputs clock information retained in the pulse oximeter 131. The acceleration sensor 504 measures accelerations. Various types of acceleration sensors, including piezoresistance, capacitance and thermally sensing types are available, but any one of these types may be employed. The display section 400 fixes the orientation of the display in accordance with the measurement result of the acceleration sensor 504. The display section 400 shows characters, numbers and the like in such an orientation that the user can view them easily, in accordance with the angle of the pulse oximeter 131.

The operation section 505 receives user's operations, for example, via a measurement start switch, a power-supply switch or operational buttons. The proximity communication unit 506 transmits data to the proximity communication unit 120. The power supply section 507 supplies power to individual sections in the pulse oximeter 131.

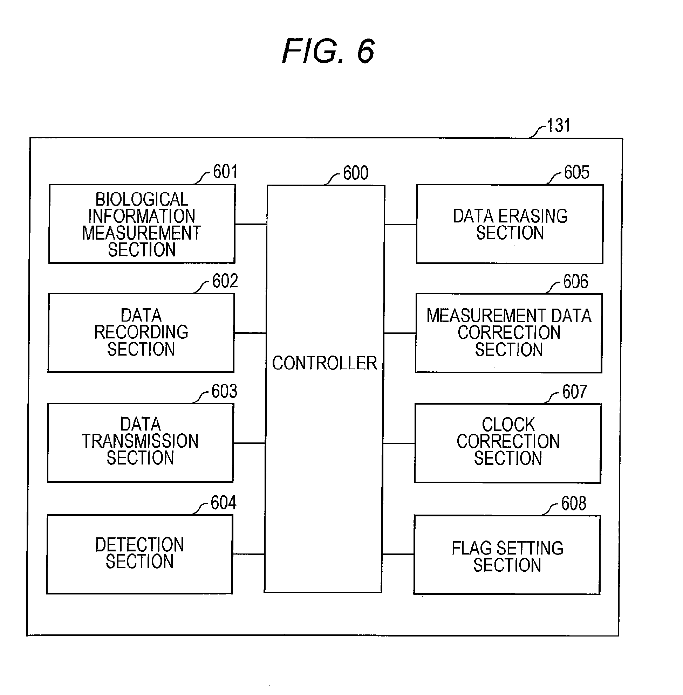

Fig. 6 is a functional block diagram of the pulse oximeter 131. This pulse oximeter 131 includes a controller 600, a biological information measurement section 601, a data storage section 602, a data transmission section 603, a detection section 604, a data erasing section 605, a measurement data correction section 606 (second data correction section), a clock correction section 607 and a flag setting section 608.

The controller 600 controls the operations of components connected thereto. The biological information measurement section 601 measures biological information on subjects, or arterial oxygen saturation (SpO2). The data storage section 602 relates measured biological information (arterial oxygen saturation (SpO2)), the measurement date and time information and the flag information in relation to one another; the measurement date and time information indicates dates and times when the biological information was measured, and the flag information indicates whether the measurement dates and times are provisional dates and times. Then, the data storage section 602 stores these pieces of information as the measurement data.

The data transmission section 603 transmits the measurement data and the clock information in the pulse oximeter 131 to the control device 110 through the near field communication. The detection section 604 detects a battery replacement event at which the clock information in the pulse oximeter 131 is reset.

The data erasing section 605 erases the measurement data from the data storage section 602 after the data transmission section 603 has transmitted the measurement data and the clock information in the pulse oximeter 131 to the control device 110.

The measurement data correction section 606 corrects the measurement date and time information contained in the measurement data which is based on the clock information in the pulse oximeter 131. This correction is based on the time discrepancy between the clock information in the pulse oximeter 131 and the clock information in the control device 110 which has been received from the control device 110 through the near field communication. After that, the measurement data correction section 606 stores the corrected measurement data in the data storage section 602.

The clock correction section 607 modifies the clock information in the pulse oximeter 131 on the basis of the clock information in the control device 110 which has been received from the control device 110 through the near field communication.

When the detection section 604 detects the battery replacement event, the flag setting section 608 sets flag information in biological information (arterial oxygen saturation (SpO2)) measured after the replacement of the battery; this flag information indicates that the measurement date and time in this biological information is a provisional date and time. When the clock information in the pulse oximeter 131 is modified to a right date and time, the flag setting section 608 sets flag information in biological information (arterial oxygen saturation (SpO2)) measured after the modification; this flag information indicates that the measurement date and time in this biological information is not a provisional date and time. It can be identified whether the clock information in the pulse oximeter 131 is modified to a right date and time, for example, on the basis of the modification of the clock information in the pulse oximeter 131 which is made by the clock correction section 607 in accordance with the clock information in the control device 110 which has been received from the control device 110 through the near field communication.

### <5. Flow of Overall Process in Biological Information Processing System>

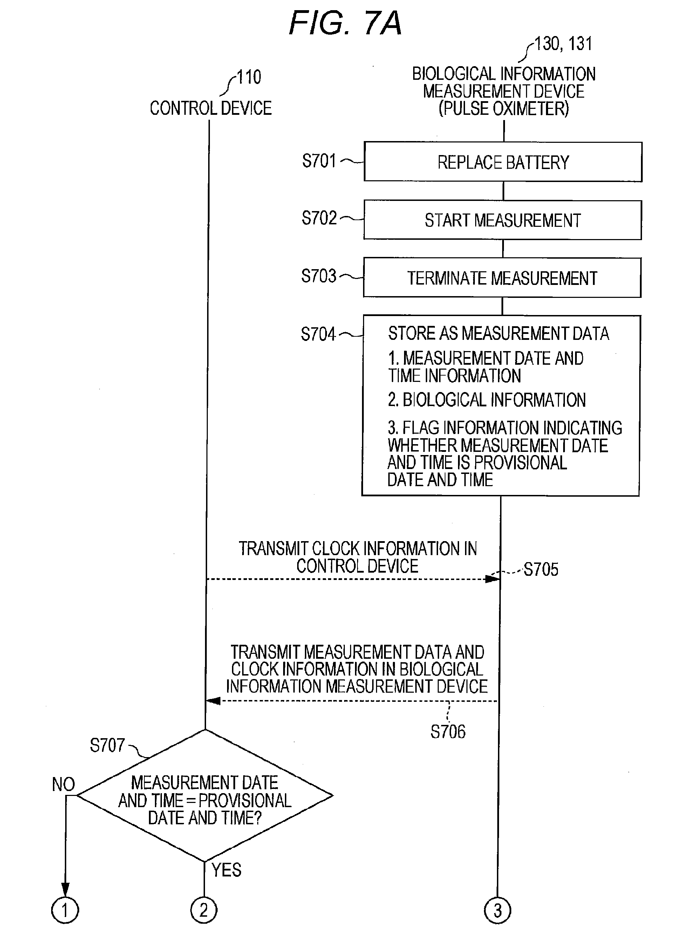

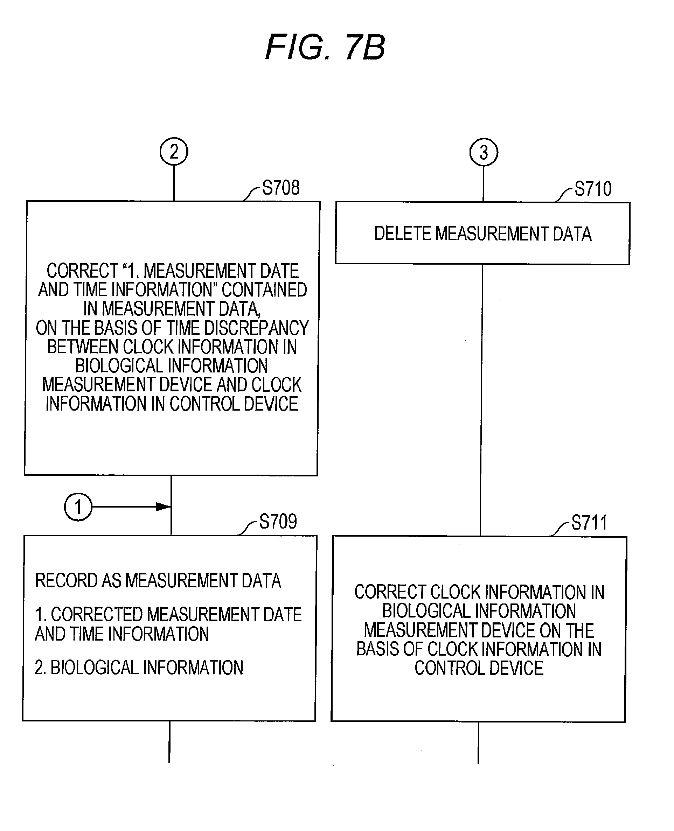

Procedures of a process performed by the biological information processing system 100 according to the first embodiment will be described with reference to Figs. 7A and 7B. The following description will be given on the premise that the process is performed between the control device 110 and the pulse oximeter 131 as an exemplary biological information measurement device 130.

At Step S701, the detection section 604 in the pulse oximeter 131 detects a battery replacement event at which the clock information in the pulse oximeter 131 is reset.

At Step S702, the biological information measurement section 601 in the pulse oximeter 131 starts measuring biological information on a subject, or arterial oxygen saturation (SpO2).

At Step S703, the biological information measurement section 601 in the pulse oximeter 131 terminates the measurement. Since the clock information in the pulse oximeter 131 has been reset in this embodiment, the flag setting section 608 in the pulse oximeter 131 sets flag information in the measured biological information, which indicates that the measurement date and time is a provisional date and time.

At Step S703, the data storage section 602 in the pulse oximeter 131 relates the measured biological information (arterial oxygen saturation (SpO2)), the measurement date and time information, and the flag information in relation to one another; the measurement date and time information indicates a date and time when the biological information was measured, and the flag information indicates whether the measurement date and time is a provisional date and time. Then, the data storage section 602 stores these pieces of information as the measurement data.

After that, a user places the pulse oximeter 131 close to or on the proximity communication unit 120 whereby data communication is made possible between the control device 110 and the pulse oximeter 131.

At Step S705, the clock information transmission section 305 in the control device 110 transmits the clock information in the control device 110 to the pulse oximeter 131 through the near field communication.

At Step S706, the data transmission section 603 in the pulse oximeter 131 transmits the measurement data and the clock information in the pulse oximeter 131 to the control device 110 through the near field communication. Note that the processes at Steps S705 and S706 may be performed in the opposite order.

At Step S707, the measurement data analysis section 302 in the control device 110 analyzes the flag information contained in the received measurement data, determining whether the measurement date and time is a provisional date and time. If it is determined that the measurement date and time is a provisional date and time (S707; YES), the processing proceeds to Step S708. If it is determined that the measurement date and time is not a provisional date and time (S707; NO), the processing proceeds to Step S709.

In this embodiment, the pulse oximeter 131 has detected the replacement of the battery at Step S701, and reset its clock information. Accordingly, the processing proceeds to Step S708. However, if the pulse oximeter 131 has not detected the replacement of the battery at Step S701 or if the pulse oximeter 131 has received from right clock information from the control device 110 and modified its clock information, the control device 110 can determine that the clock information in the pulse oximeter 131 is right and accordingly stores the measurement data in relation to flag information indicating that the measurement date and time is not provisional date and time at Step S704. In this case, the processing proceeds to Step S709.

At Step S708, the measurement data correction section 303 in the control device 110 corrects the measurement date and time information contained in the measurement data, on the basis of the time discrepancy between the clock information in the control device 110 and the clock information in the pulse oximeter 131 which has been received from the pulse oximeter 131 through the near field communication. As a result, this measurement date and time information becomes a right measurement date and time.

At Step S709, the data recording section 304 in the control device 110 relates the biological information contained in the measurement data to the corrected measurement date and time information that the measurement data correction section 303 has corrected, and records both pieces of information as the corrected measurement data. If the measurement date and time is determined not to be a provisional date and time at Step S707, the data recording section 304 records the biological information contained in the measurement data that the control device 110 has received at Step S706 and its measurement date and time information.

After the data transmission section 603 has transmitted the measurement data to the control device 110 through the near field communication at Step S706, the data erasing section 605 in the pulse oximeter 131 deletes this measurement data at Step S710.

At Step S711, the clock correction section 607 in the pulse oximeter 131 modifies the clock information therein on the basis of the clock information in the control device 110 which the pulse oximeter 131 has received from the control device 110 through the near field communication at Step S706. Note that the processes at Steps S710 and S711 may be performed in the opposite order. Then, the process in Figs. 7A and 7B is terminated.

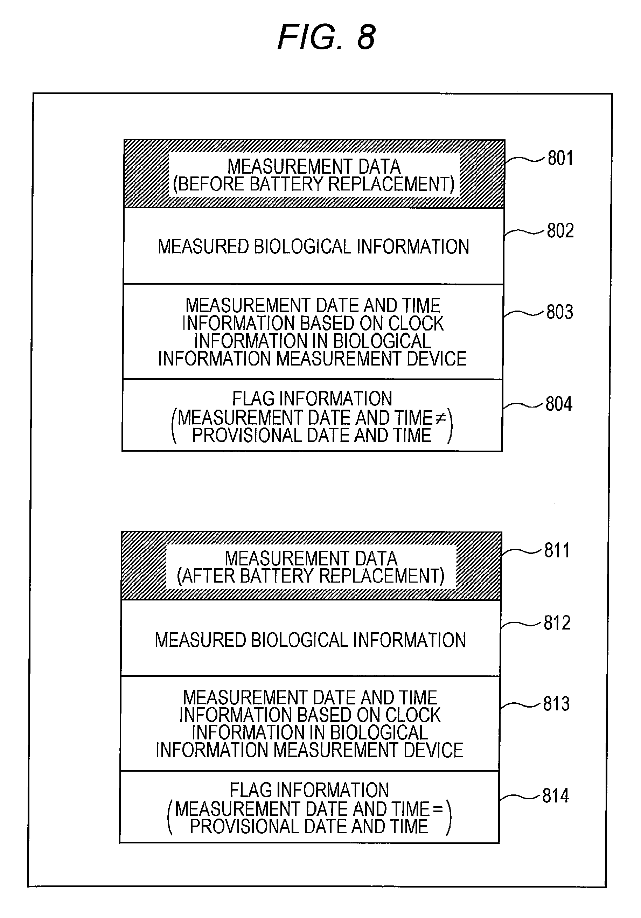

Fig. 8 is a view illustrating an exemplary configuration of the measurement data. Measurement data 801 is data that is measured before the replacement of the battery, for example, at Step S701 in Figs. 7A and 7B; measurement data 811 is data that is measured after the replacement of the battery.

The measurement data 801 contains biological information 802, measurement date and time information 803, and flag information 804; the biological information 802 is measured by the biological information measurement device 130, the measurement date and time information 803 is based on the clock information in the biological information measurement device 130, and the flag information 804 indicates that the measurement date and time is not a provisional date and time. The measurement data 811 contains biological information 812, measurement date and time information 813, and flag information 814; the biological information 812 is measured by the biological information measurement device 130, the measurement date and time information 813 is based on the clock information in the biological information measurement device 130, and the flag information 814 indicates that the measurement date and time is a provisional date and time.

When the measurement data is transmitted at Step S706 in Fig. 7A, if both the measurement data 801 that has been measured as a right date and time before the replacement of the battery at Step S701 and the measurement data 811 that has been measured as a provisional date and time after the replacement of the battery are present, these pieces of data are transmitted to the control device 110. Even in this case, the control device 110 analyzes the pieces of flag information. Then, the control device 110 can record the measurement data 801 as it is, and in turn record the measurement data 811 after correcting its measurement date and time.

In this embodiment, as described above, a biological information measurement device relates measured biological information on subjects, measurement date and time information regarding the biological information, and flag information in relation to one another; the measurement date and time information is based on clock information in the biological information measurement device, and the flag information indicates whether the measurement date and time is a provisional date and time. Then, the biological information measurement device stores these pieces of information as measurement data. A control device receives the measurement data from the biological information measurement device and analyzes the flag information contained in this measurement data. Depending on this analyses result, the control device corrects the measurement date and time in the measurement data, on the basis of the clock information in the biological information measurement device which has received from the biological information measurement device. As a result, this measurement date and time becomes a right date and time. Then, the control device records the corrected measurement date and time.

According to this embodiment, when clock information in a biological information measurement device is reset and displaced from right clock information, a control device corrects measurement date and time information contained in measurement data. This enables a measurement result of biological information measurement device to be managed appropriately.

Moreover, the biological information measurement device deletes measurement data after transmitting it to the control device. This enables efficient utilization of the memory in the biological information measurement device. The biological information measurement device modifies its clock information on the basis of right clock information received from the control device. Therefore, the measurement data acquired from subsequent measurement will be proper data that requires no date and time correction.

### [Second Embodiment]

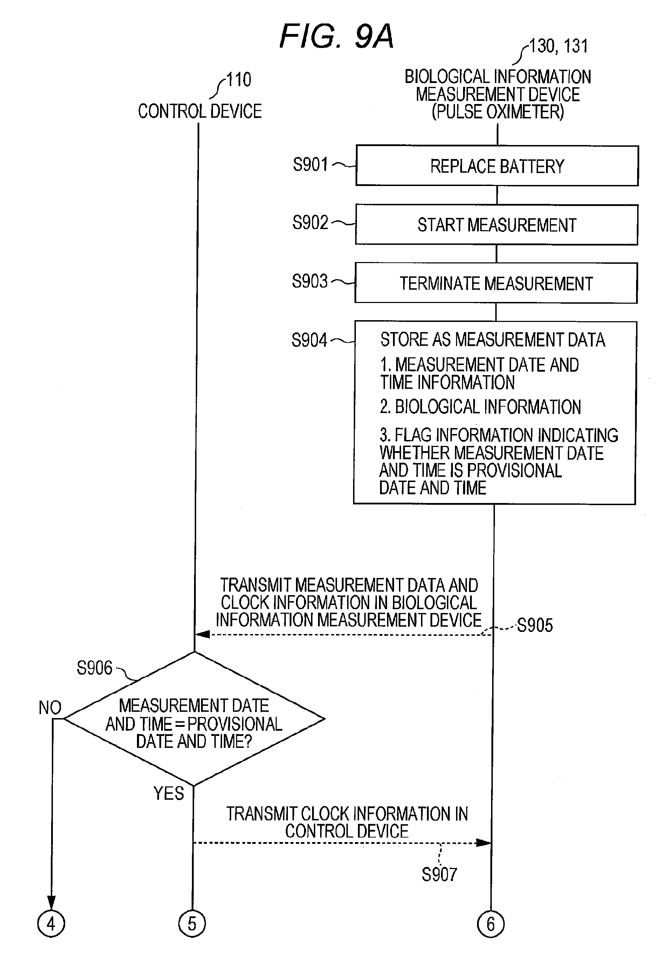

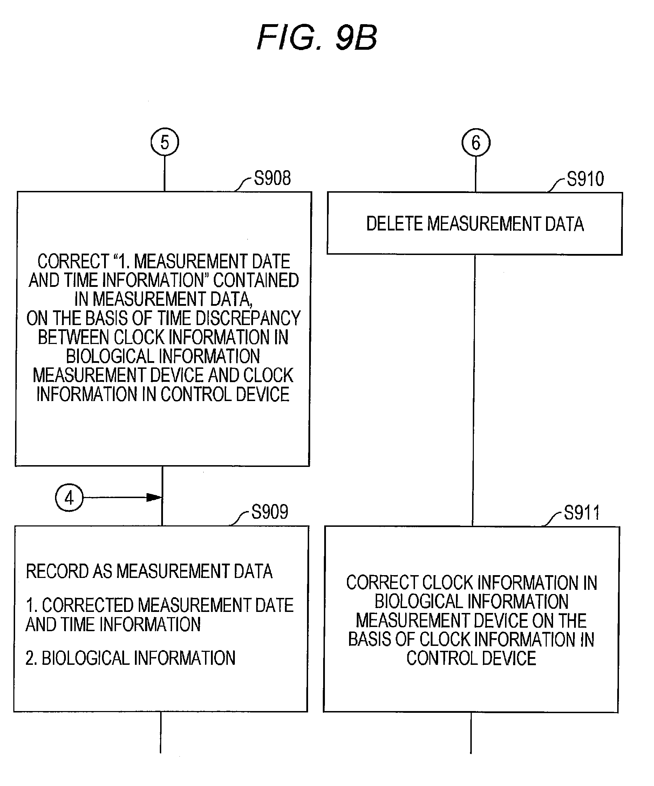

Procedures of a process performed by a biological information processing system 100 according to a second embodiment will be described with reference to Figs. 9A and 9B. Like the first embodiment, the following description will be given on the premise that the process is performed between a control device 110 and a pulse oximeter 131 as an exemplary biological information measurement device 130. Components that are identical to those in the first embodiment are denoted by the same reference characters. Processes that are identical to those having been described in the first embodiment with reference to Figs. 7A and 7B will not be described.

Processes at Steps S901 to S905 are the same as those at Steps S701 to S704 and S706, respectively, in Fig. 7A.

A process at Steps S906 is the same as that at Step S707.

If a measurement date and time is determined to be a provisional date and time at Step S906, a clock information transmission section 305 in the control device 110 transmits clock information in the control device 110 to the pulse oximeter 131 through near field communication at Step S907.

Subsequent processes at Steps S908 to S911 are the same as those at Steps 708 to S711, respectively, in Fig. 7B.

If the biological information measurement device 130 transmits multiple pieces of measurement data to the control device 110 at Step S905, the control device 110 only has to transmit its clock information to the biological information measurement device 130 when a provisional flag is set in at least one of the pieces of measurement data.

In this embodiment, as described above, a control device transmits its clock information for use in modifying clock information in a biological information measurement device only when a measurement date and time is a provisional date and time. Thus, if clock information in the biological information measurement device is right, no process of transmitting clock information is required. This makes it possible to shorten the time needed to transmit data.

### [Third Embodiment]

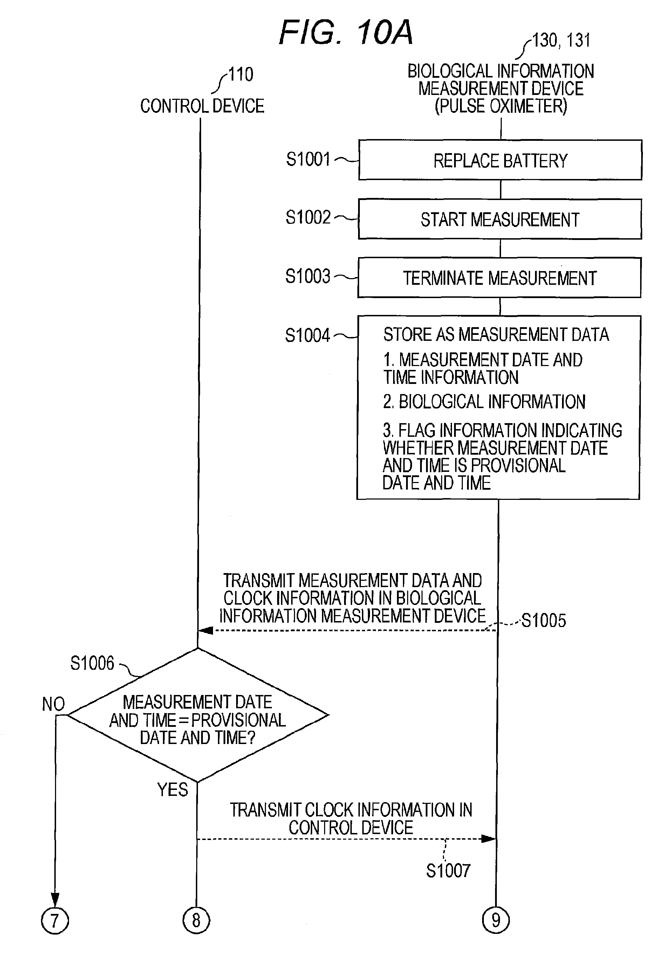

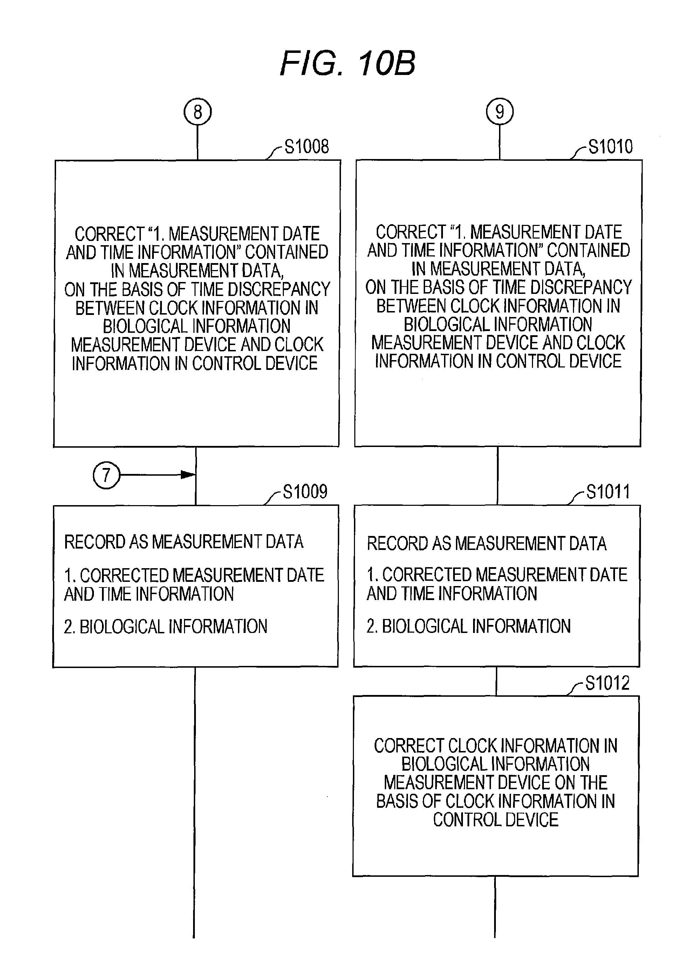

Procedures of a process performed by a biological information processing system 100 according to a third embodiment will be described with reference to Figs. 10A and 10B. Like the second embodiment, the following description will be given on the premise that the process is performed between a control device 110 and a pulse oximeter 131 as an exemplary biological information measurement device 130. Components that are identical to those in the first embodiment are denoted by the same reference characters. Processes that are identical to those having been described in the second embodiment with reference to Figs. 9A and 9B will not be described.

Processes at Steps S1001 to S1009 are the same as those at Steps S901 to S909, respectively, in Figs. 9A and 9B.

At Step S1010, a measurement data correction section 606 in the pulse oximeter 131 corrects measurement date and time information contained in measurement data so that it becomes a right measurement date and time, on the basis of the time discrepancy between clock information in the pulse oximeter 131 and clock information in the control device 110 which the pulse oximeter 131 has received from the control device 110 through near field communication at Step S1007.

At Step S1011, a data storage section 602 in the pulse oximeter 131 relates biological information contained in the measurement data to the corrected measurement date and time information corrected by the measurement data correction section 606. Then, the data storage section 602 stores both pieces of information as corrected measurement data.

A process at Step S1012 is the same as that at Step S911.

In this embodiment, as described above, a control device transmits its clock information for use in modifying clock information in a biological information measurement device only when a measurement date and time is a provisional date and time. Thus, if clock information in the biological information measurement device is right, no process of transmitting clock information is required. This makes it possible to shorten the time needed to transmit data. Moreover, a biological information measurement device also corrects measurement date and time information in measurement data so that it becomes a right date and time, and then stores it. It is thus possible to ensure pieces of measurement history data arranged in proper time sequence in the biological information measurement device.

### [Fourth Embodiment]

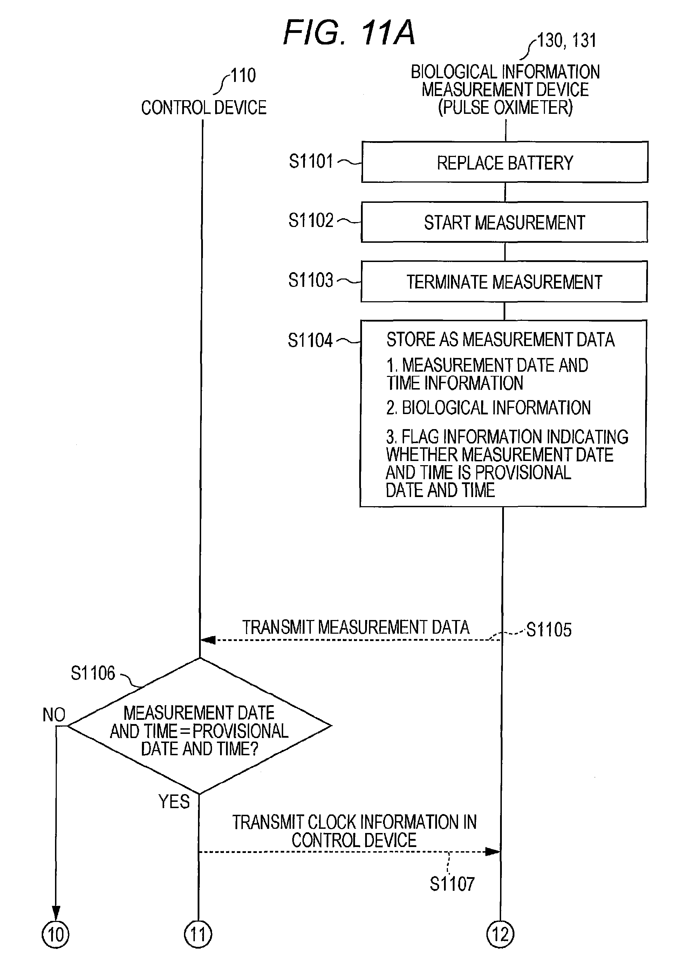

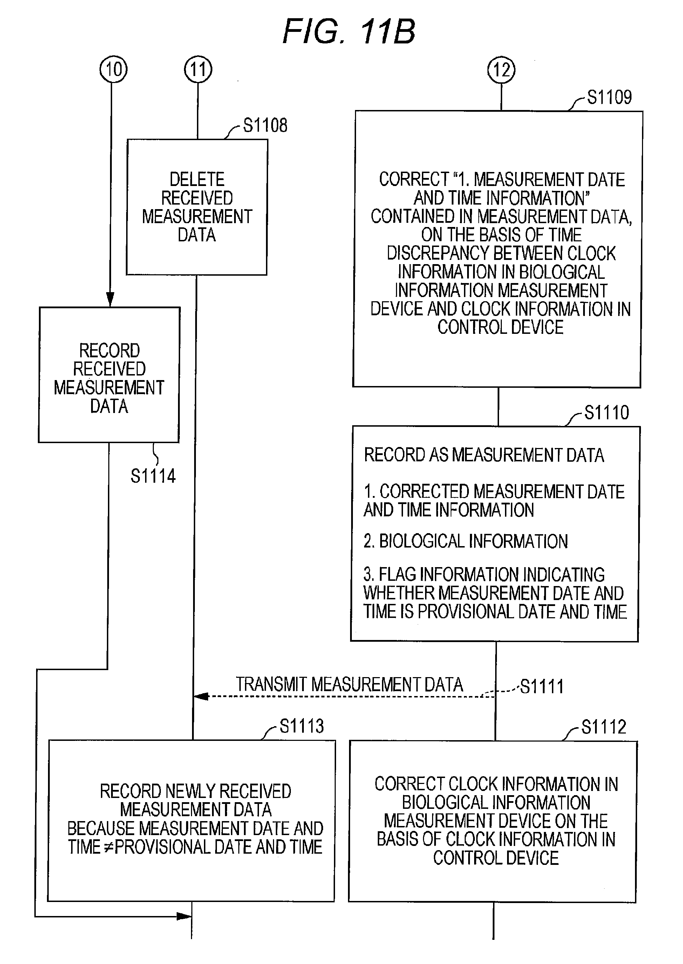

Procedures of a process performed by a biological information processing system 100 according to a fourth embodiment will be described with reference to Figs. 11A and 11B. Like the second embodiment, the following description will be given on the premise that the process is performed between a control device 110 and a pulse oximeter 131 as an exemplary biological information measurement device 130. Components that are identical to those in the first embodiment are denoted by the same reference characters. Processes that are identical to those having been described in the second embodiment with reference to Figs. 9A and 9B will not be described.

Processes at Steps S1101 to S1107 are the same as those at Steps S901 to S907, respectively, in Fig. 9A. At Step S1105, however, a data transmission section 603 in the pulse oximeter 131 transmits measurement data to the control device 110 through near field communication. Note that in this case it is not necessarily necessary to transmit clock information in the pulse oximeter 131.

Since the analysis has showed that a measurement date and time is a provisional date and time at Step S1106, a data erasing section 306 in the control device 110 deletes the received measurement data without recording it at Step S1108.

At Step S1109, a measurement data correction section 606 in the pulse oximeter 131 corrects measurement date and time information contained in the measurement data, on the basis of the time discrepancy between clock information in the pulse oximeter 131 and clock information in the control device 110 that the pulse oximeter 131 has received from the control device 110 through the near field communication at Step S1107. As a result, this measurement date and time information becomes a right measurement date and time.

At Step S1110, a data storage section 602 in the pulse oximeter 131 relates the corrected measurement date and time information, biological information and flag information; the corrected measurement date and time information is corrected by the measurement data correction section 606, the biological information is contained in the measurement data, and the flag information indicates whether the measurement date and time is a provisional date and time. Then, the data storage section 602 stores these pieces of information as corrected measurement data. In this case, the related flag information indicates that the measurement date and time is not a provisional date and time.

At Step S1111, the data transmission section 603 in the pulse oximeter 131 transmits the corrected measurement data to the control device 110 through the near field communication.

At Step S1112, a clock correction section 607 in the pulse oximeter 131 corrects the clock information in the pulse oximeter 131, on the basis of the clock information in the control device 110 that the pulse oximeter 131 has received from the control device 110 through the near field communication at Step S1107. Note that the process at Step S1112 may be performed at any given timing after the clock information in the control device 110 has been received at Step S1107.

At Step S1113, a data recording section 304 in the control device 110 records newly received measurement data (because measurement date and time ≠ provisional date and time).

At Step S1114, the data recording section 304 in the control device 110 records the measurement data received at Step S1105 without deleting it (because measurement date and time ≠ provisional date and time).

In this embodiment, as described above, a control device transmits its clock information for use in modifying clock information in a biological information measurement device only when a measurement date and time is a provisional date and time. Thus, if clock information in the biological information measurement device is right, no process of transmitting clock information is required. This makes it possible to shorten the time needed to transmit data.

In this embodiment, a biological information measurement device receives clock information in a control device from the control device. Then, it corrects a measurement date and time and transmits the corrected measurement data to the control device. In this case, the control device need not perform a process of correcting measurement data. Therefore, it is possible to utilize the resource in the control device effectively.

The present invention is not limited to the embodiments described above and can be modified and varied in various ways without departing from its spirit and scope. Therefore, the claims described below are attached in order to disclose the scope of the present invention.

## Claims

1. A biological information processing system comprising:
a biological information measurement device measuring biological information on a subject; and
a control device managing the biological information,
the biological information measurement device including
storage means for relating the biological information, measurement date and time information, and flag information to one another and storing these pieces of information as measurement data, the measurement date and time information indicating a date and time when the biological information is measured, the flag information indicating whether the measurement date and time is a provisional date and time when a clock is not adjusted yet, and
data transmission means for transmitting the measurement data and measurement-side clock information possessed by the biological information measurement device to the control device, and
the control device including
analysis means for analyzing the flag information contained in the measurement data, the measurement data being received from the biological information measurement device,
data correction means for, when the analysis conducted by the analysis means shows that the measurement date and time is the provisional date and time, correcting the measurement date and time information on the basis of a time discrepancy between the measurement-side clock information and control-side clock information possessed by the control device; and
recording means for relating the biological information to corrected measurement date and time information corrected by the data correction means and recording both pieces of information as corrected measurement data.

2. The biological information processing system according to claim 1, wherein
the control device further includes clock information transmission means for transmitting the control-side clock information to the biological information measurement device.

3. The biological information processing system according to claim 2, wherein
when the analysis conducted by the analysis means shows that the measurement date and time is the provisional date and time, the clock information transmission means transmits the control-side clock information to the biological information measurement device.

4. The biological information processing system according to any one of claims 1 to 3, wherein
the biological information measurement device further includes data erasing means for, when the data transmission means transmits the measurement data and the measurement-side clock information to the control device, deleting the measurement data stored in the storage means.

5. The biological information processing system according to any one of claims 1 to 3, wherein
the biological information measurement device further includes second data correction means for, when the measurement date and time is the provisional date and time, correcting the measurement date and time information on the basis of the time discrepancy between the measurement-side clock information and the control-side clock information received from the control device, and
the storage means relates the biological information to corrected measurement date and time information corrected by the second data correction means and storing both pieces of information again as corrected measurement data.

6. The biological information processing system according to any one of claims 1 to 5, wherein
the biological information measurement device further includes
detection means for detecting replacement of a battery in the biological information measurement device; and
flag setting means for, when the detection means detects the replacement of the battery, setting flag information indicating that a measurement date and time of the biological information measured after the replacement of the battery is the provisional date and time.

7. The biological information processing system according to claim 6, wherein
the biological information measurement device further includes clock correction means for correcting the measurement-side clock on the basis of the control-side clock information received from the control device, and
when the clock correction means corrects the measurement-side clock, the flag setting means sets flag information indicating that the measurement date and time information in the biological information measured after the correction of the measurement-side clock is not the provisional date and time.

8. A biological information measurement device that measures biological information on a subject, the biological information measurement device comprising:
storage means for relating the biological information, measurement date and time information, and flag information to one another and storing these pieces of information as measurement data, the measurement date and time information indicating a date and time when the biological information is measured, the flag information indicating whether the measurement date and time is a provisional date and time; and
data transmission means for transmitting the measurement data and measurement-side clock information possessed by the biological information measurement device to the control device.

9. A control device that manages biological information on a subject which is measured by a biological information measurement device, the control device comprising:
receiving means for receiving measurement data and measurement-side clock information from the biological information measurement device, the measurement data being related to the biological information, measurement date and time information, and flag information, the measurement-side clock information being possessed by the biological information measurement device, the measurement date and time information indicating a date and time when the biological information is measured, the flag information indicating whether the measurement date and time is a provisional date and time;
analysis means for analyzing the flag information contained in the measurement data;
data correction means for, when the analysis conducted by the analysis means shows that the measurement date and time is the provisional date and time, correcting the measurement date and time information on the basis of a time discrepancy between the measurement-side clock information and control-side clock information possessed by the control device; and
recording means for relating the biological information to corrected measurement date and time information corrected by the data correction means and recording both pieces of information as corrected measurement data.

10. A method of controlling a biological information processing system, the biological information processing system including a biological information measurement device that measures biological information on a subject and a control device that manages the biological information, the method comprising:
a storing step of, by using storage means in the biological information measurement device, relating the biological information, measurement date and time information, and flag information to one another and storing these pieces of information as measurement data, the measurement date and time information indicating a date and time when the biological information is measured, the flag information indicating whether the measurement date and time is a provisional date and time when a clock is not adjusted yet;
a data transmitting step of, by using data transmission means in the biological information measurement device, transmitting the measurement data and measurement-side clock information possessed by the biological information measurement device to the control device;
an analyzing step of, by using analysis means in the control device, analyzing the flag information contained in the measurement data, the measurement data being received from the biological information measurement device;
a data correcting step of, by using data correction means in the biological information measurement device, when the analysis conducted at the analyzing step shows that the measurement date and time is the provisional date and time, correcting the measurement date and time information on the basis of a time discrepancy between the measurement-side clock information and control-side clock information possessed by the control device; and
recording step of, by using recording means in the biological information measurement device, relating the biological information to corrected measurement date and time information corrected at the data correcting step and recording both pieces of information as corrected measurement data.

11. A method of controlling a biological information measurement device that measures biological information on a subject, the biological information measurement device including storage means and data transmission means, the method comprising:
a storing step of, by using the storage means, relating the biological information, measurement date and time information, and flag information to one another and storing these pieces of information as measurement data, the measurement date and time information indicating a date and time when the biological information is measured, the flag information indicating whether the measurement date and time is a provisional date and time when a clock is not adjusted yet; and
a data transmitting step of, by using the data transmission means, transmitting the measurement data and measurement-side clock information possessed by the biological information measurement device to the control device.

12. A method of controlling a control device that manages biological information on a subject which is measured by a biological information measurement device, the control device including receiving means, analysis means, data correction means and recording means, the method comprising:
receiving step of receiving measurement data and measurement-side clock information from the biological information measurement device by using the receiving means, the measurement data being related to the biological information, measurement date and time information, and flag information, the measurement-side clock information being possessed by the biological information measurement device, the measurement date and time information indicating a date and time when the biological information is measured, the flag information indicating whether the measurement date and time is a provisional date and time when a clock is not adjusted yet;
an analyzing step of analyzing the flag information contained in the measurement data by using the analysis means;
a data correcting step of, by using the data correction means, when the analysis conducted at the analyzing step shows that the measurement date and time is the provisional date and time, correcting the measurement date and time information on the basis of a time discrepancy between the measurement-side clock information and control-side clock information possessed by the control device; and
a recording step of, by using the recording means, relating the biological information to corrected measurement date and time information corrected at the data correcting step and recording both pieces of information as corrected measurement data.

13. A storage medium that stores a program causing a computer to perform the steps in the method of controlling a biological information measurement device according to claim 11.

14. A storage medium that stores a program causing a computer to perform the steps in the method of controlling a control device according to claim 12.
